# DEMANDE DE BREVET EUROPEEN

(11) **EP 1 155 680 A1**
(43) Date de publication de la demande: **21.11.2001**
(21) Numéro de dépôt: 01401148.0
(22) Date de dépôt: 03.05.2001
(51) Int. Cl.: A61K 7/13

(54) **Compositions pour la teinture d'oxydation des fibres kératiniques comprenant au moins la 5-méthyl pyrazolo-(1,5a)-pyrimidine-3,7-diamine comme base d'oxydation ; procédés de teinture de mise en oeuvre**

(30) Priorité: 19.05.2000 FR 0007120
(71) Demandeur: L'OREAL, 75008 Paris (FR)
(72) Inventeur: Kravtchenko, Sylvain, 92600 Asnieres (FR); Lagrange, Alain, 77700 Coupvray (FR)
(74) Mandataire: Fevrier, Murielle

(57) **Abrégé**

L'invention a pour objet une composition pour la teinture d'oxydation des fibres kératiniques, et en particulier des fibres kératiniques humaines telles que les cheveux, comprenant au moins comme base d'oxydation la 5-méthyl pyrazolo-[1,5-a]-pyrimidine-3,7-diamine ; ladite composition ne contenant pas de coupleur du type N-(2-hydroxybenzène)-carbamate ou du type N-(2-hydroxybenzène)-urée et les procédés de teinture d'oxydation la mettant en oeuvre.

## Description

L'invention a pour objet une composition pour la teinture d'oxydation des fibres kératiniques, et en particulier des fibres kératiniques humaines telles que les cheveux, comprenant au moins comme base d'oxydation la 5-méthyl pyrazolo-[1,5-a]-pyrimidine-3,7-diamine ; ladite composition ne contenant pas de coupleur du type N-(2-hydroxybenzène)-carbamate ou du type N-(2-hydroxybenzène)-urée et les procédés de teinture d'oxydation la mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Il a déjà été proposé, notamment dans la demande de brevet DE 4029324, d'utiliser certains dérivés de pyrazolo-[1,5-a]-pyrimidine, pouvant être substitués par des radicaux alkyls en C₁-C₄ en position 4, 5 et/ou 6, comme coupleurs pour la teinture d'oxydation des fibres kératiniques.

Il a été proposé aussi dans la demande de brevet DE 4133957, d'utiliser certains dérivés de pyrazolo-[1,5-a]-pyrimidine appartenant à la famille des tétrahydro pyrazolo-1,5-a]-pyrimidine comme précurseurs de colorant d'oxydation pour la teinture d'oxydation des fibres kératiniques.

On a préconisé dans la demande de brevet EP-A-0847271 d'utiliser comme bases d'oxydation pour la teinture d'oxydation des fibres kératiniques certains dérivés de pyrazolo-[1,5-a]-pyrimidine tels que la pyrazolo-[1,5-a]-pyrimidine-3,7-diamine. Ces dérivés ont une bonne aptitude à colorer les fibres kératiniques de manière tenace et puissante ainsi qu'un bon profil toxicologique. Cependant, les dérivés de pyrazolo-[1,5-a]-pyrimidine présentent pour la plupart des problèmes de solubilité dans les supports de teinture, ce qui limite considérablement leur niveau d'incorporation dans les formulations et la possibilité d'obtenir des colorations dans l'intensité recherchée.

Or, la demanderesse vient maintenant de découvrir, de façon totalement inattendue et surprenante, qu'il était possible d'obtenir des teintures, capables de conduire à des colorations puissantes, particulièrement chromatiques et brillantes, peu sélectives, et présentant d'excellentes propriétés de résistances aux diverses agressions que peuvent subir les fibres kératiniques en utilisant à titre de base d'oxydation une pyrazolo-[1,5-a]-pyrimidine particulière présentant une solubilité sensiblement supérieure à celle des pyrazolo-[1,5-a]-pyrimidine citées précédemment.

Il s'agit de la 5-méthyl pyrazolo-[1,5-a]-pyrimidine-3,7-diamine de structure suivante : 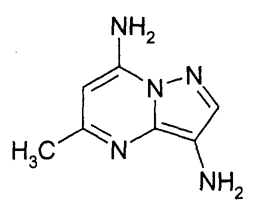 ainsi que ses sels d'addition avec un acide ou avec une base.

Ces découvertes sont à la base de la présente invention.

L'invention a donc pour premier objet une composition pour la teinture d'oxydation des fibres kératiniques, et en particulier des fibres kératiniques humaines telles que les cheveux, caractérisée par le fait qu'elle contient, dans un milieu approprié pour la teinture au moins comme base d'oxydation la 5-méthyl pyrazolo-[1,5-a]-pyrimidine-3,7-diamine de structure : 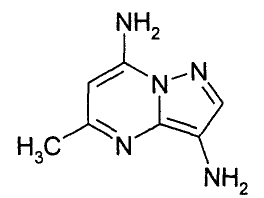 ainsi que ses sels d'addition avec un acide ou avec une base; ladite composition ne contenant pas de coupleur du type N-(2-hydroxybenzène)-carbamate ou du type N-(2-hydroxybenzène)-urée.

Comme indiqué précédemment, les colorations obtenues avec la composition de teinture d'oxydation conforme à l'invention sont puissantes, particulièrement brillantes et chromatiques . Elles permettent d'atteindre en particulier des nuances rouges exemptes ou contenant très peu de bleu ou de jaune. Elles présentent de plus d'excellentes propriétés de résistance vis à vis de l'action des différents agents extérieurs (lumière, intempéries, lavage, ondulation permanente, transpiration, frottements).

L'invention a également pour objet un procédé de teinture d'oxydation des fibres kératiniques mettant en oeuvre cette composition tinctoriale.

La 5-méthyl pyrazolo-[1,5-a]-pyrimidine-3,7-diamine représente de préférence de 0,001 à 10 % en poids environ du poids total de la composition tinctoriale, et encore plus préférentiellement de 0,05 à 6 % en poids environ de ce poids.

La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et des bases hétérocycliques différentes de la 5-méthyl pyrazolo-[1,5-a]-pyrimidine-3,7-diamine utilisée conformément à l'invention.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2630438, et leurs sels d'addition.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-amino phényl) tétra-méthylènediamine, la N,N'-bis-(β-hydroxyéthyl) N,N'-bis-(4-aminophényl) tétraméthylènediamine, la N,N'-bis-(4-méthylaminophényl) tétraméthylène-diamine, la N,N'-bis-(éthyl) N,N'-bis-(4'-amino, 3'-méthylphényl) éthylènediamine, et leurs sels d'addition.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxy-méthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut plus particulièrement citer à titre d'exemple, les dérivés pyridiniques, les dérivés pyrimidiniques, les dérivés pyrazoliques différents des dérivés de pyrazolo-[1,5-a]-pyrimidine de formule (I) utilisés conformément à l'invention, tels qu'en particulier les 4,5-diamino pyrazoles, et leurs sels d'addition.

Lorsqu'elles sont utilisées, ces bases d'oxydation additionnelles représentent de préférence de 0,0005 à 12% en poids environ du poids total de la composition tinctoriale, et encore plus préférentiellement de 0,005 à 6% en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols, les dérivés mono ou polyhydroxylé du naphtalène et les coupleurs hétérocycliques tels que par exemple les dérivés indoliques ou pyridiniques et leurs sels d'addition.

Ces coupleurs sont plus particulièrement choisis parmi le 2-méthyl 5-amino phénol, le 5-N-(β-hydroxyéthyl)amino 2-méthyl phénol, le 6-chloro-2méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydoxy benzomorpholine et leurs sels d'addition.

Lorsqu'ils sont présents ces coupleurs représentent de préférence de 0,0001 à 10% en poids environ du poids total de la composition tinctoriale et encore plus préférentiellement de 0,005 à 5% en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates. Les sels d'addition avec une base utilisables dans le cadre des compositions tinctoriales de l'invention (bases d'oxydation et coupleurs) sont notamment ceux obtenus avec la soude, la potasse, l'ammoniaque ou les amines.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant. La composition tinctoriale peut éventuellement contenir des catalyseurs d'oxydation, afin d'accélérer le processus d'oxydation.

Selon une première forme de mise en oeuvre du procédé de l'invention, la coloration des fibres peut être effectuée sans addition d'un agent oxydant, au seul contact de l'oxygène de l'air.

Selon une deuxième forme de mise en oeuvre du procédé de l'invention, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES 1 à 11 (Teintures en milieu alcalin)

On réalise les formulations les formulations colorantes suivantes :

### Support de teinture commun :

| | |
|---|---|
| 5-méthyl pyrazolo-[1,5-a]-pyrimidine-3,7-diamine | 0.708 g |
| Coupleur | yg |
| Alcool benzylique | 2g |
| Polyéthylène glycol 8 OE | 3g |
| Ethanol | 18g |
| Alkyl (C₈-C₁₀)polyglucoside en solution aqueuse à 60% de matière active tamponné par du citrate d'ammonium vendu sous la dénomination ORAMIX CG110 par SEPPIC | 5g M.A. |
| Ammoniaque à 20% de NH₃ | 10g |
| Métabisulfite de sodium | 0.205 g |
| Séquestrant | q.s. |
| Eau déminéralisée | q.s.p. 100 g |

| **TEINTURES A PH ALCALIN** | | |
|---|---|---|
| **Exemples** | **Coupleur associé à la 5-méthyl pyrazolo-[1,5-a]-pyrimidine-3,7- diamine** | **Quantité introduite dans la formule (en g%) - valeur de y** |
| Exemple 1 | Résorcine | 0,330 |
| Exemple 2 | Métaaminophénol | 0,327 |
| Exemple 3 | 2-méthyl-5-aminophénol | 0,369 |
| Exemple 4 | 2-méthylrésorcine | 0,372 |
| Exemple 5 | 6-hydroxyindole | 0,399 |
| Exemple 6 | Dichlorhydrate de 2,4-diaminophénoxyéthanol | 0,723 |
| Exemple 7 | 6-hydroxybenzomorphine | 0,453 |
| Exemple 8 | 2- amino-3-hydroxypyridine | 0,330 |
| Exemple 9 | Chlorure de 5-(N-méthyl-3'-pyridyl)méthyl-1-méthyl-4-hydroxyindole | 0,866 |
| Exemple 10 | 3,6-diméthyl-1H-pyrazolo[5,1-c][1,2,4] triazole | 0,408 |
| Exemple 11 | 6-chloro-2méthyl-5-aminophénol | 0,582 |

Au moment de l'emploi, on a mélangé chaque composition tinctoriale 1 à 11, poids pour poids, avec une solution d'eau oxygénée à 20 volumes (6% en poids) dont le pH a été ajusté à environ 2,5 avec de l'acide orthophosphorique.

Le mélange est appliqué sur des cheveux gris à 90% de blancs, permanentés ou non, à raison de 300 mg pour 30mg de cheveux, pendant 30 min.

Les cheveux sont ensuite rincés, lavés avec un shampooing standard et séchés.

Les mèches de cheveux ont été teintées dans les nuances figurant dans le tableau ci-dessous :

| **NUANCES OBTENUES A PH ALCALIN** | |
|---|---|
| Exemple 1 | Acajou cuivré |
| Exemple 2 | Acajou cuivré |
| Exemple 3 | Cuivré rouge |
| Exemple 4 | Cuivré |
| Exemple 5 | Marron doré |
| Exemple 6 | Violine |
| Exemple 7 | Irisé rouge |
| Exemple 8 | Irisé violine |
| Exemple 9 | Violet |
| Exemple 10 | Cuivré doré |
| Exemple 11 | Rouge cuivré |

La 5-méthyl pyrazolo-[1,5-a]-pyrimidine-3,7-diamine permet donc d'obtenir une large palette de couleur à pH alcalin.

### EXEMPLES 12 à 22 (Teintures en milieu neutre)

On réalise les formulations les formulations colorantes suivantes :

### Support de teinture commun :

| | |
|---|---|
| 5-méthyl pyrazolo-[1,5-a]-pyrimidine-3,7-diamine | 0.708 g |
| Coupleur | yg |
| Alcool benzylique | 2g |
| Polyéthylène glycol 8 OE | 3g |
| Ethanol | 18g |
| Alkyl (C₈-C₁₀) polyglucoside en solution aqueuse à 60% de matière active tamponné par du citrate d'ammonium vendu sous la dénomination ORAMIX CG110 par SEPPIC | 5g M.A. |
| Tampon phosphate (K₂HPO₄ 1.5M / KH₂PO₄ 1M) | 10g |
| Métabisulfite de sodium | 0.205g |
| Séquestrant | q.s. |
| Eau déminéralisée | q.s.p. 100g |

| **TEINTURES A PH = 7** | | |
|---|---|---|
| **Exemples** | **Coupleur associé à la 5-méthyl pyrazolo-[1,5-a]-pyrimidine-3,7- diamine** | **Quantité introduite dans la formule (en g%) - valeur de y** |
| Exemple 12 | Résorcine | 0,330 |
| Exemple 13 | Méta-aminophénol | 0,327 |
| Exemple 14 | 2-méthyl-5-aminophénol | 0,369 |
| Exemple 15 | 2-méthylrésorcine | 0,372 |
| Exemple 16 | 6-hydroxyindole | 0,399 |
| Exemple 17 | Dichlorhydrate de 2,4-diaminophénoxyéthanol | 0,723 |
| Exemple 18 | 6-hydroxybenzomorphine | 0,453 |
| Exemple 19 | 2- amino-3-hydroxypyridine | 0,330 |
| Exemple 20 | Chlorure de 5-(N-méthyl-3'-pyridyl)méthyl-1-méthyl-4-hydroxyindole | 0,866 |
| Exemple 21 | 3,6-diméthyl-1H-pyrazolo[5,1-c][1,2,4] triazole | 0,408 |
| Exemple 22 | 6-chloro-2méthyl-5-aminophénol | 0,582 |

Au moment de l'emploi, on a mélangé chaque composition tinctoriale 12 à 22, poids pour poids, avec une solution d'eau oxygénée à 20 volumes (6% en poids) dont le pH a été ajusté à environ 2,5 avec de l'acide orthophosphorique.

Le mélange est appliqué sur des cheveux gris à 90% de blancs, permanentés ou non, à raison de 300 mg pour 30mg de cheveux, pendant 30 min.

Les cheveux sont ensuite rincés, lavés avec un shampooing standard et séchés.

Les mèches de cheveux ont été teintées dans les nuances figurant dans le tableau ci-dessous :

| **NUANCES OBTENUES A PH 7** | |
|---|---|
| Exemple 12 | Acajou cuivré |
| Exemple 13 | Acajou cuivré |
| Exemple 14 | Cuivré rouge |
| Exemple 15 | Irisé cuivré |
| Exemple 16 | Marron |
| Exemple 17 | Violine |
| Exemple 18 | Irisé rouge |
| Exemple 19 | Cendré irisé |
| Exemple 20 | Violet |
| Exemple 21 | Cuivré doré |
| Exemple 22 | Rouge cuivré |

La 5-méthyl pyrazolo-[1,5-a]-pyrimidine-3,7-diamine permet donc d'obtenir une large palette de couleur à pH neutre.

## Revendications

1. Composition pour la teinture d'oxydation des fibres kératiniques, comprenant, dans un milieu approprié pour la teinture au moins comme base d'oxydation la 5-méthyl pyrazolo-[1,5-a]-pyrimidine-3,7-diamine de structure : 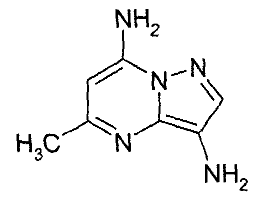 et/ou ses sels d'addition avec un acide ou avec une base ; ladite composition ne contenant pas de coupleur du type N-(2-hydroxybenzène)-carbamate ou du type N-(2-hydroxybenzène)-urée.

2. Composition selon la revendication 1, **caractérisée par le fait que** la 5-méthyl pyrazolo-[1,5-a]-pyrimidine-3,7-diamine ou ses sels représente de 0,001 à 10% en poids du poids total de la composition tinctoriale.

3. Composition selon la revendication 2, **caractérisée par le fait que** la 5-méthyl pyrazolo-[1,5-a]-pyrimidine-3,7-diamine ou ses sels représente de 0,05 à 6 % en poids du poids total de la composition tinctoriale.

4. Composition selon l'une quelconque des revendications 1 à 3, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

5. Composition selon l'une quelconque des revendications 1 à 4, **caractérisée par le fait qu'**elle présente un pH compris entre 3 et 12.

6. Composition selon l'une quelconque des revendications 1 à 5, **caractérisée par le fait qu'**elle renferme au moins une base d'oxydation additionnelle choisie parmi les paraphénylènediamines, les bis-phénylalkylènediamines, les paraaminophénols, les ortho-aminophénols et des bases hétérocycliques différentes de la 5-méthyl pyrazolo-[1,5-a]-pyrimidine-3,7-diamine.

7. Composition selon la revendication 6, **caractérisée par le fait que** la ou les bases d'oxydation additionnelles représentent de 0,0005 à 12% en poids du poids total de la composition tinctoriale.

8. Composition selon l'une quelconque des revendications 1 à 7, **caractérisée par le fait qu'**elle renferme au moins un coupleur et/ou au moins un colorant direct.

9. Composition selon la revendication 8, **caractérisée par le fait que** le ou les coupleurs sont choisis parmi les métaphénylènediamines, les méta-aminophénols, les métadiphénols, les dérivés mono ou polyhydroxylés du naphtalène et les coupleurs hétérocycliques et leurs sels d'addition avec un acide.

10. Composition selon l'une quelconque des revendications 8 à 9, **caractérisée par le fait que** le ou les coupleurs représentent de 0,0001 à 10 % en poids du poids total de la composition tinctoriale.

11. Composition selon l'une quelconque des revendications 1 à 10, **caractérisée par le fait que** les sels d'addition avec un acide sont choisis parmi les chlorhydrates, les bromhydrates, les sulfates et les tartrates, les lactates et les acétates et les sels d'addition avec une base sont choisis parmi ceux obtenus avec la soude, la potasse, l'ammoniaque ou les amines.

12. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 11, pendant un temps suffisant pour développer la coloration désirée, soit à l'air, soit à l'aide d'un agent oxydant, éventuellement en présence de catalyseurs d'oxydation.

13. Procédé selon la revendication 12, **caractérisé par le fait que** la coloration est révélée au seul contact de l'oxygène de l'air.

14. Procédé selon I revendication 12 **caractérisé par le fait que** l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

15. Procédé selon les revendications 12 ou 14, **caractérisé par le fait que** l'agent oxydant est choisi parmi le peroxyde d'hydrogène, le peroxyde d'urée, les bromates de métaux alcalins, les persels tels que les perborates et persulfates.

16. Dispositif à plusieurs compartiments, ou "kit" de teinture à plusieurs compartiments, dont un premier compartiment renferme une composition tinctoriale telle que définie à l'une quelconque des revendications 1 à 11 et un second compartiment renferme une composition oxydante.
